(19) Europäisches Patentamt
European Patent Office
Office européen des brevets

(11) Publication number: **0 155 812 B1**

(12) **EUROPEAN PATENT SPECIFICATION**

(45) Date of publication of patent specification: **11.09.91**

(51) Int. Cl.$^5$: **A61K 6/08**, C08F 2/50

(21) Application number: **85301705.1**

(22) Date of filing: **12.03.85**

(54) **Dental compositions.**

(30) Priority: **16.03.84 JP 51883/84**

(43) Date of publication of application:
**25.09.85 Bulletin 85/39**

(45) Publication of the grant of the patent:
**11.09.91 Bulletin 91/37**

(84) Designated Contracting States:
**DE FR GB IT NL**

(56) References cited:
**EP-A- 0 136 186**
**EP-A- 0 150 952**
**FR-A- 2 337 890**

(73) Proprietor: **KURARAY CO., LTD.**
**1621 Sakazu**
**Kurashiki-City Okayama Prefecture 710(JP)**

(72) Inventor: **Hino, Kenichi**
**1660, Sakazu**
**Kurashiki-city(JP)**
Inventor: **Yamauchi, Junichi**
**21-7, Hachiojicho**
**Kurashiki-city(JP)**
Inventor: **Nishida, Koji**
**c/o Ranpuryo 1652-1, Sakazu**
**Kurashiki-city(JP)**

(74) Representative: **Crampton, Keith John Allen et al**
**D. YOUNG & CO. 10 Staple Inn**
**London WC1V 7RD(GB)**

**EP 0 155 812 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the polymerization and hardening, by irradiation with visible light, of dental compositions that adhere to teeth and to dental restorative materials such as metals, ceramics and polymers and that comprise monomers containing acidic groups in the molecule.

In dental treatment in recent years, one very important development is the technique for adhesion to the teeth or to metals. Vinyl monomers containing acidic groups play a large role in enhancing the adhesive property of dental compositions: when such monomers are used, adhesion to the teeth or metals is sufficient for dental treatment. Examples of such monomers include methacrylic acid esters containing acidic groups such as phosphoric or phosphonic acid diester groups, phosphoric or phosphonic acid mono ester groups, pyrophosphoric acid groups, phosphinic acid groups, carboxyl groups, acid anhydride groups, and acid halide groups.

In recent dental techniques, attention has been focused upon methods for photohardening instead of conventional methods using redox polymerization initiators, as a method for hardening dental compositions. For this reason, attempts to incorporate photopolymerization initiators into polymerizable monomer-containing compositions have been made in order to carry out photohardening. Among known photohardening techniques, materials such as α-diketones are used as sensitizers and amines are used as accelerators in U.S. Patent Specification US-A-4 071 424, benzoin alkyl ethers or benzyls are used as sensitizers and organic peroxides are used as accelerators in Japanese Patent Application OPI 102/80, and α-diketonesare used as sensitizers and organic peroxides are used as accelerators in U.S. Patent Specification US-A-4 459 193.

However, catalyst systems using organic peroxides or tertiary amines as photohardening accelerators for acidic-group-containing materials require irradiation for a longer and impracticable period, compared with conventional photopolymerizable compositions containing (meth)acrylic esters instead of acidic-group-containing monomers.

French Patent Specification FR-A-2337890 discloses a photopolymerisable composition comprising: (a) a vinyl monomer; (b) an accelerator that contains at least one mercapto group and no aldehyde group in the molecule; and (c) a photosensitizer that is an α-diketone or a quinone.

In accordance with the present invention, a dental composition comprises a vinyl monomer containing at least one acidic group in the molecule and an initiator capable of photopolymerizing the monomer by visible light, characterised in that the initiator comprises camphorquinone as a photosensitizer, together with an accelerator that contains at least one mercapto group in the molecule and does not contain an aldehyde group in the molecule.

The invention also extends to a kit for restoration of a tooth comprising a combination of (1) a dental composition comprising a vinyl monomer containing at least one acidic group in the molecule and an initiator and an accelerator as defined above, and (2) a dental filling composition comprising a polymerizable monomer, an initiator capable of polymerizing the monomer and a filler.

The term "acidic group" as used herein, in addition to including narrowly defined acidic groups such as

-COOH, 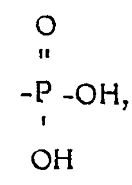 , 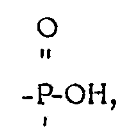 , 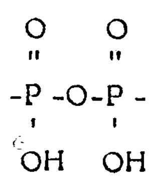 and 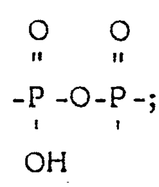 ;

also includes acid anhydride groups such as

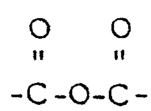

and acid halide groups such as

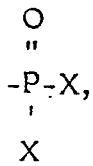
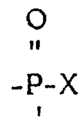
or
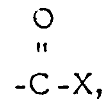

where X represents F, Cl, Br or I.

Specific examples of the monomers include the following compounds:

(1) Monomers having a

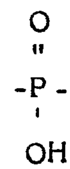

group:

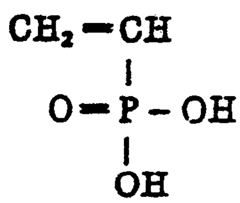
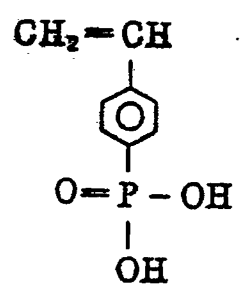

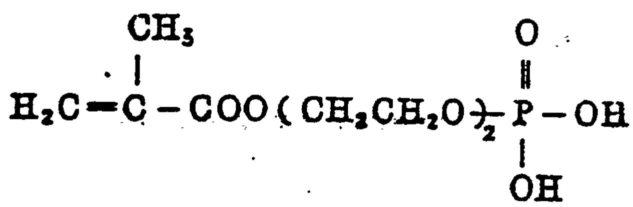

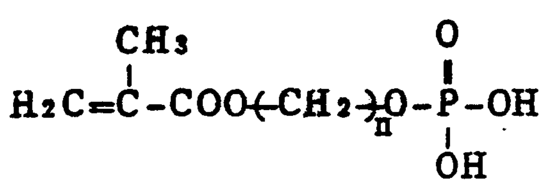

(where n is an integer of 2 to 40)

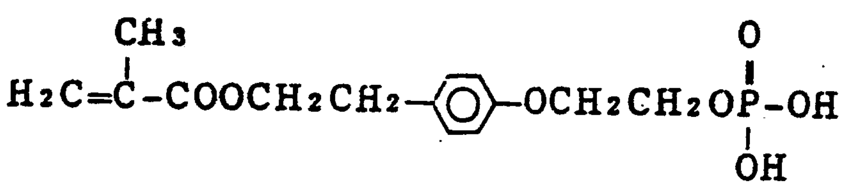

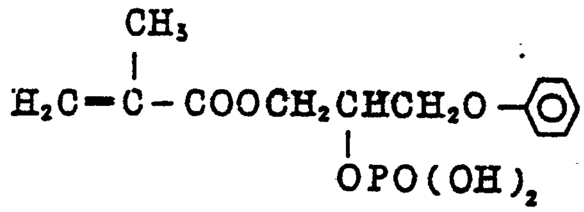

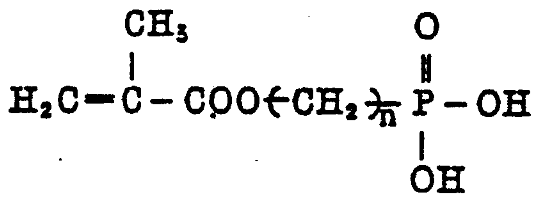

(where n is an integer of 2 to 40)

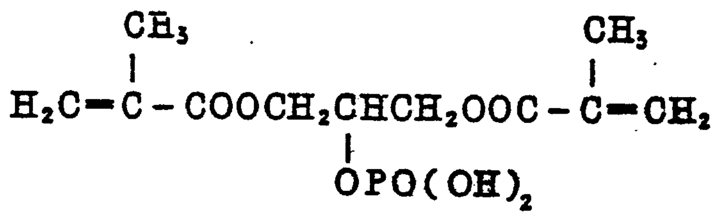

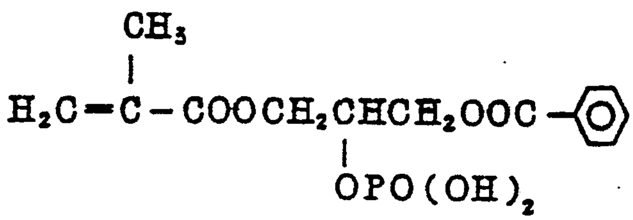

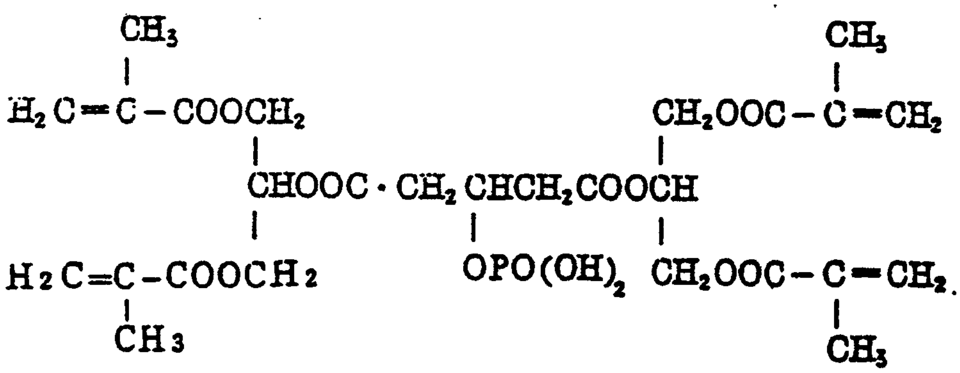

(2) Monomer having a

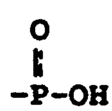

group:

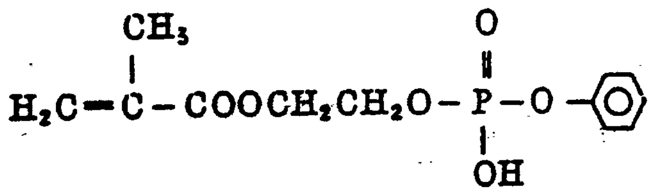

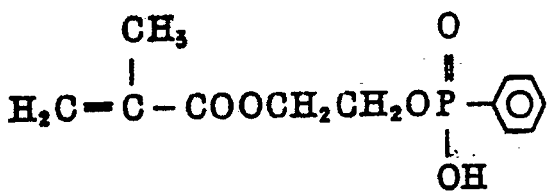

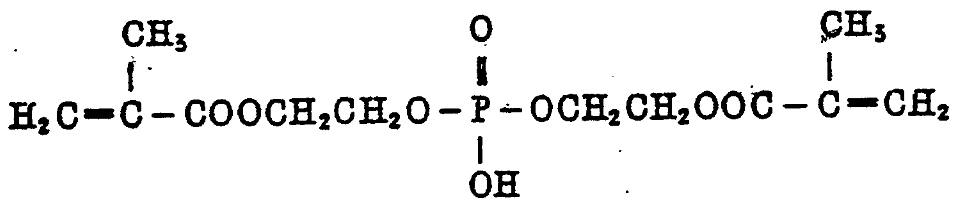

(3) Monomer having a

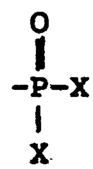

group:

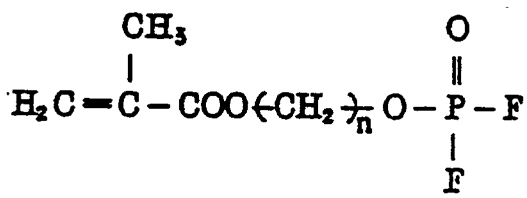

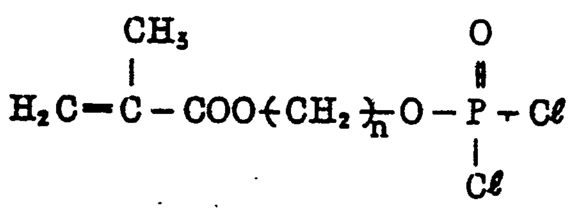

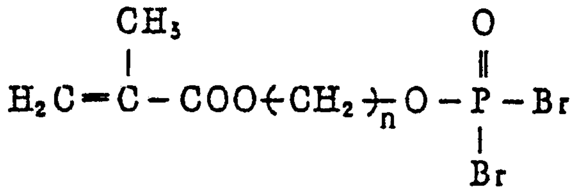

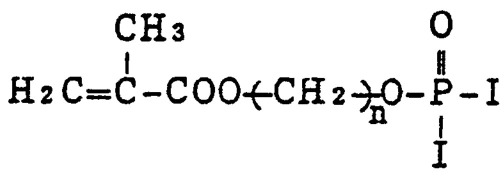

(where n is an integer of 2 to 20)

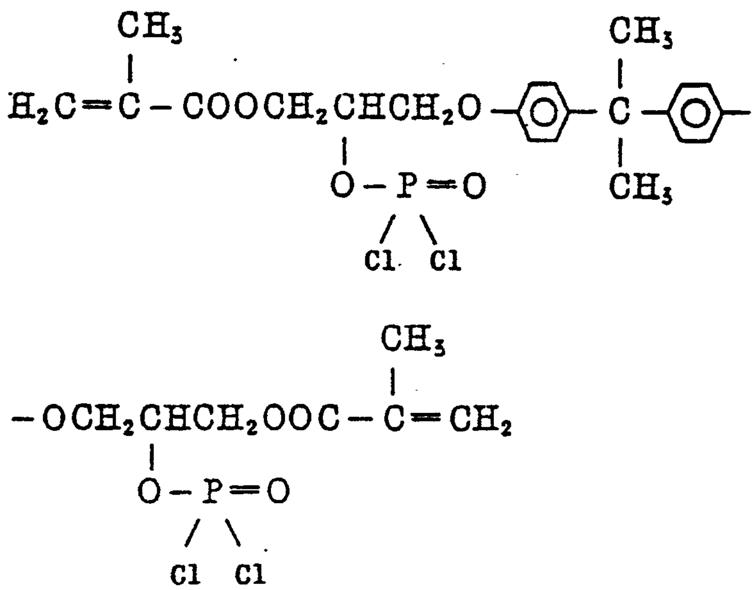

(4) Momoner having a

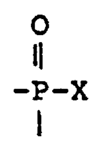

group:

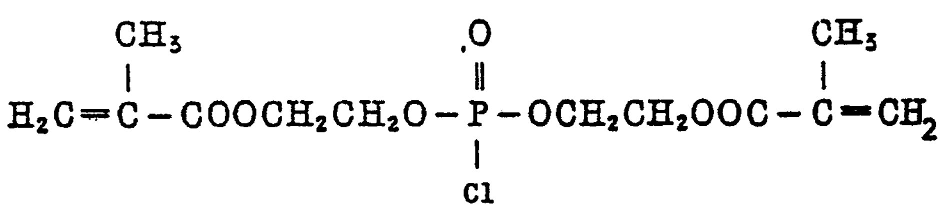

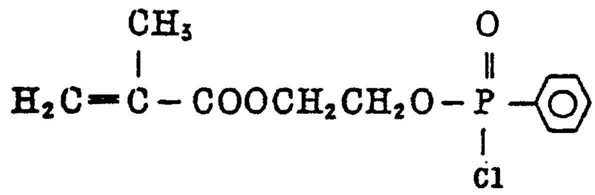

(5) Monomer having a

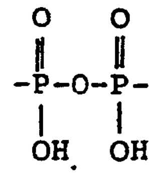

group:

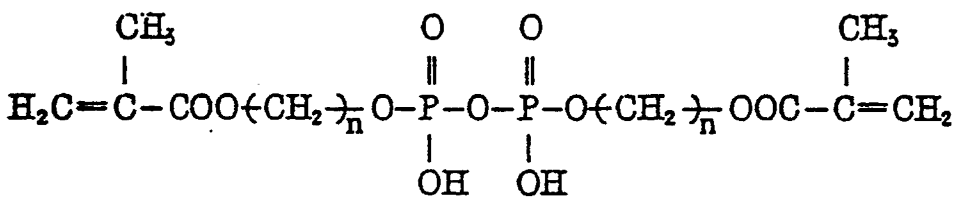

(where n is an integer of 2 to 20)
(6) Monomer having

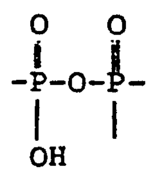

group:

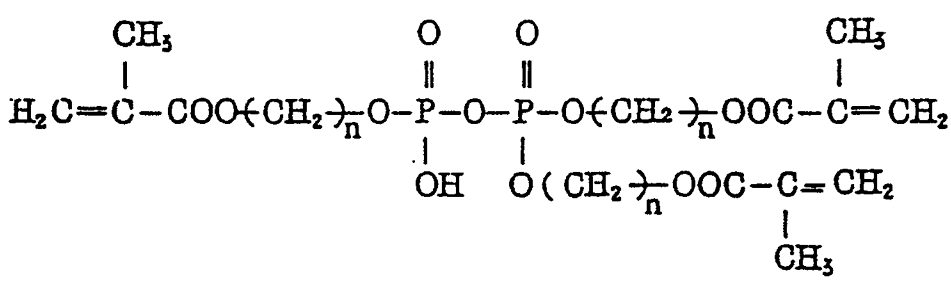

(where n is an integer of 2 to 20)
(7) Monomer having a -COOH group:

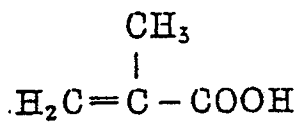
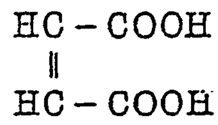
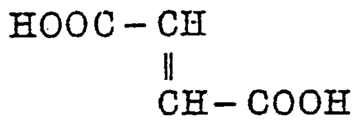
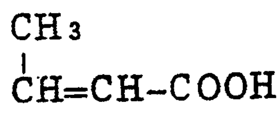
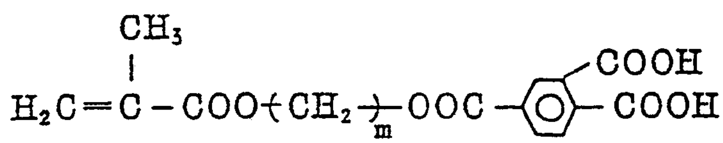

(where m is an integer of 2 to 12)

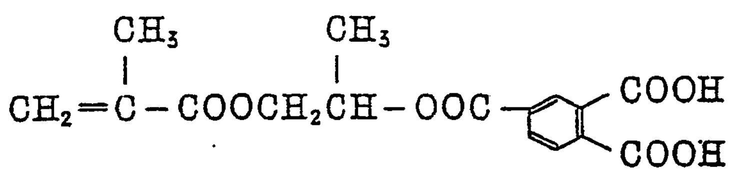
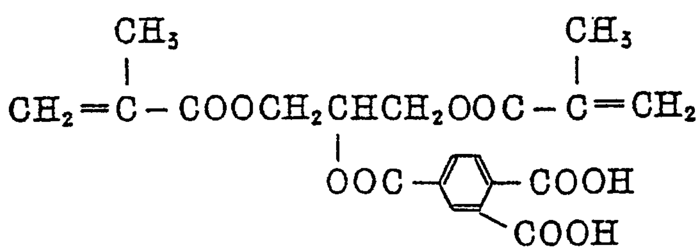
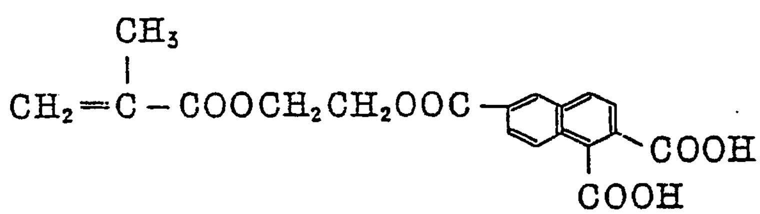
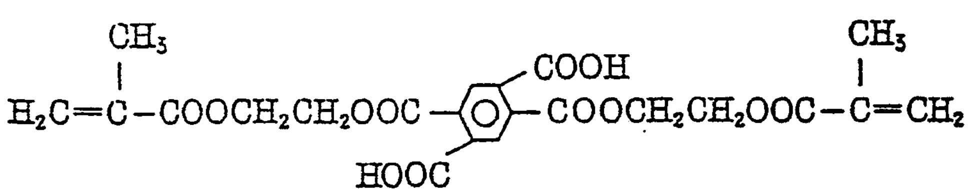
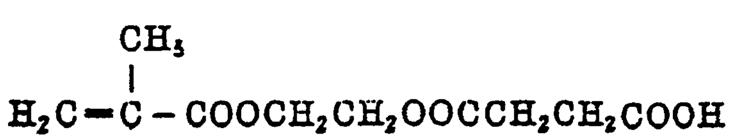

(8) Monomer having a

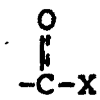

group:

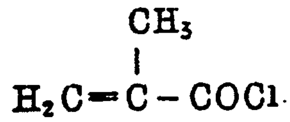

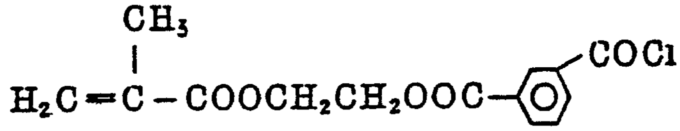

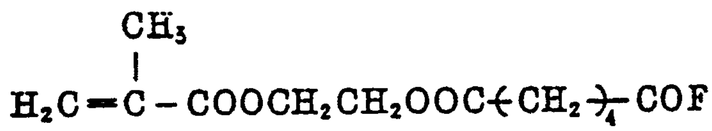

(9) Monomer having a

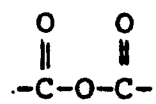

group:

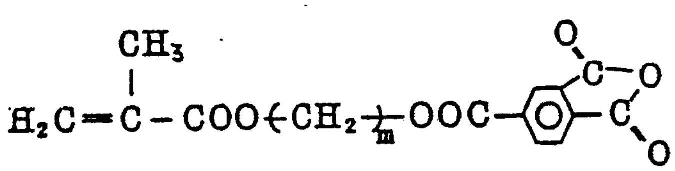

(where m is an integer of 2 to 12)

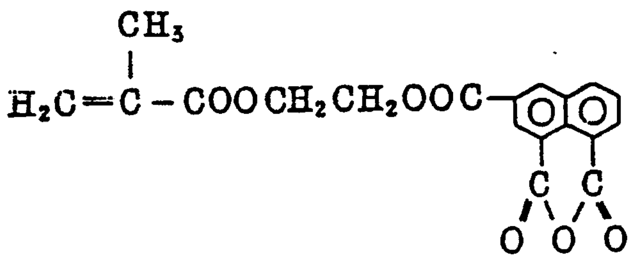

In the present invention, the monomers may be composed only of monomers having the aforesaid acidic groups but usually copolymers later described are involved. The monomers containing the acidic groups usually make up more than 0.5 wt% of the total monomers in order to retain the adhesive property. Vinyl monomers copolymerizable with the vinyl monomers containing acidic groups include esters of α-cyanoacrylic acid, (meth)acrylic acid, urethane (meth)acrylic acid, crotonic acid, cinnamic acid, sorbic acid,

maleic acid and itaconic acid with alcohols or diols; (meth)acrylamides such as N-isobutylacrylamide; vinyl esters of carboxylic acids such as vinyl acetate; vinyl ethers such as butyl vinyl ether; mono-N-vinyl compounds such as N-vinylpyrrolidone; and styrene and its derivatives. Esters of monofunctional or polyfunctional (meth)acrylic acids and urethane (meth)acrylic acid esters as described below are preferred.

(i) Monofunctional Esters

Methyl (meth)acrylate, *n*- or *iso*-propyl (meth)acrylate, *n*- or *iso*- or *t*-butyl (meth)acrylate, 2-hydroxyethyl methacrylate (hereafter referred to as HEMA),

(ii) Bifunctional Esters

Esters shown by the general formula:

$$CH_2=\overset{R_1}{\overset{|}{C}}-CO(O-CH_2-CH_2)_nO-CO-\overset{R_1}{\overset{|}{C}}=CH_2$$

where n is an integer of from 1 to 14 and $R_1$ is hydrogen or a methyl group; such as di(meth)acrylates of ethylene glycol, diethylene glycol, triethylene glycol (hereafter referred to as 3G), tetraethylene glycol, dodecaethylene glycol and tetradecaethylene glycol, glycerine di(meth)acrylate, 2,2'-bis[*p*-(γ-methacryloxy-β-hydroxypropoxy)phenyl]propane (hereinafter referred to as Bis-GMA), bisphenol A dimethacrylate, neopentylglycol di(meth)acrylate (hereafter referred to as NPG), 2,2'-di(4-methacryloxypolyethoxyphenyl)-propane containing 2 to 10 ethoxy groups in one molecule, and 1,2-bis(3-methacryloxy-2-hydroxypropoxy)-butane.

(iii) Tri- or higher functional Esters

Trimethylolpropane tri(meth)acrylate and pentaerythritol tetra(meth)acrylate.

(iv) Urethane (Meth)acrylate Type

Examples are the reaction products of 2 moles of (meth)acrylate monomers having a hydroxyl group and 1 mole of diisocyanates, and the reaction products of urethane prepolymers having NCO at the terminals and (meth)acrylate monomers having a hydroxyl group. Examples of such reaction products include those having the formula:

$$CH_2=\overset{R_1}{\overset{|}{C}}-\underset{\underset{O}{\|}}{C}-O-R_2-O-\underset{\underset{O}{\|}}{C}-NH-R_3-NH-\underset{\underset{O}{\|}}{C}-O-R_2-O-\underset{\underset{O}{\|}}{C}-\overset{R_1}{\overset{|}{C}}=CH_2$$

where $R_1$ is hydrogen or a methyl group, $R_2$ is an alkylene group and $R_3$ is an organic residue. Specific examples include the reaction product of 2,2,4-trimethylhexamethylenediisocyanate with oxypropyl methacrylate, which is described in Japanese Patent Publication 36960/76, and the reaction product of a urethane prepolymer having isocyanates at both terminals with 2-oxyethyl methacrylate, which is described in Japanese Patent Publication 33867/80. The tetrafunctional monomers disclosed in U.S. Patent Specification US-A-4 386 912 may also be used.

The photosensitizer used in the present invention is camphorquinone (common name of 1,7,7-trimethylbicyclo[2,2,1]heptane-2,3-dione), which has clear absorption at 380 to 500 nm in the UV and visible absorption spectrum. It is used in a range of 0.01 to 5 wt % based on the polymerisable monomers.

The accelerator used in the present invention is a compound containing at least one mercapto group, and no aldehyde group, in the molecule and includes the following compounds:

(a) compounds represented by general formula: B - SH, where B represents a $C_{1-20}$ aliphatic hydrocarbon or $C_{6-20}$ aromatic hydrocarbon group optionally having alkyl, aryl, hydroxyl, alkoxy, carboxyl, alkoxycarbonyl, acyl, dialkylamino, amino, mercapto, halogen, carbamoyl, or nitro substitution in which the alkyl, aryl, alkoxy, alkoxycarbonyl, acyl and dialkyl amino groups have up to 20 carbon atoms;

(b) compounds represented by general formula:

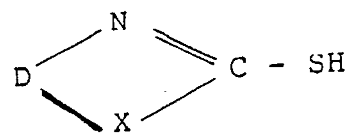

where D forms a 5- or 6-membered ring together with the

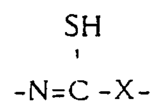

residue and is bound through a single bond or a double bond or is composed of 2 or 3 carbon atoms forming a part of one aromatic ring, or is composed of one nitrogen atom bound to one carbon atom; and X represents -O-, -S-,

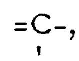

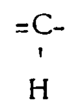

or NR where R is H or $C_{1-4}$ alkyl;

(c) compounds represented by general formula:

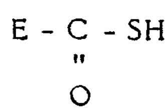

where E represents a phenyl group, which optionally contains a $C_{1-20}$ alkyl substituent, or a $C_{1-20}$ alkyl group.

Specific examples of the photosensitizers include the following compounds.

Compounds shown in (a) above: alkanethiols such as 1-octadecanethiol, 1-dodecanethiol and 1-eicosanethiol; cycloalkanethiols such as cyclohexanethiol; arylthiols such as thiophenol, naphthalene-2-thiol, 2-naphthacenethiol and 4-cholanthrenethiol (1,2-dihydrobenz[h]aceanthrylene-4-thiol); aralkanethiols such as phenylmethanethiol, naphthacen-2-ylmethanethiol and 1,2-dihydrobenz[h]aceanthrylen-4-ylmethanethiol; alkarylthiols such as thiocresol, butylbenzenethiol, p-eicosylthiophenol and p-phytylthiophenol; hydroxy-substituted thiols such as 2-mercaptoethanol and 3-mercapto-1,2-propanediol; alkoxy-substituted thiols such as methoxybenzenethiol and 2-methoxyethanethiol; carboxy-substituted thiols such as 2-mercaptoacetic acid, 3-mercaptopropionic acid and thiosalicyclic acid; alkoxycarbonyl-substituted thiols such as ethyl 3-mercaptopropionate; acyl-substituted thiols such as 4-mercaptoacetophenone; dialkylamino-substituted thiols such as N,N-dimethylaminoethanethiol hydrochloride and N,N-diisopropylaminoethanethiol hydrochloride; amino-substituted thiols such as 4-aminothiophenol; polythiols such as 1,4-butanedithiol and 1,9-nonanedithiol; halogen-substuted thiols such as chlorothiophenol, bromothiophenol and fluorothiophenol, carbamoyl-substituted thiols such as 4-acetamidothiophenol; and nitro-substituted thiols such as 4-nitrothiophenol.

Compounds represented by (b) above: 2-mercaptothiazoline, 2-mercaptopyridine, 2-mercaptoquinoline, 2-mercaptoimidazole, 2-mercapto-1-methylimidazole, 1H-1,2,4-triazole-3-thiol, 2-mercaptobenzoxazole, 2-mercaptobenzothiazole, 2-mercaptobenzimidazole, 2-mercapto-1-ethylbenzimidazole and 2-mercapto-1-butylbenzimidazole.

Compounds represented by (c) above: thiobenzoic acid, thiopropionic acid, heneicosanethioic S-acid and p-eicosylbenzenecarbothioic S-acid.

In addition, furfuryl thiol and methylfurfuryl thiol may be used.

These accelerators are used in a range of 0.1 to 10 wt% based on the polymerizable monomers, after the optimum concentration has been determined depending upon polymerization system. In the present invention, the polymerization initiators may be composed of the above-mentioned two components (photosensitizers and accelerators).

The compositions of the present invention may contain, in addition to the above-mentioned polymerizable monomers and photopolymerizable initiators, a variety of fillers depending upon the purpose of the composition. The fillers may be organic, e.g. methyl poly(meth)acrylates, ethyl poly(meth)acrylates, and materials obtained by coating an inorganic filler as described below with polymers, or may be inorganic, which may be powdery, fibrous or thin pieces composed of such materials as silicon dioxide, alumina, various glasses, ceramics, clay minerals such as diatomaceous earth, kaolin, montmorillonite or Japanese acid clay, synthetic zeolite, mica, calcium fluoride, calcium phosphate, barium sulphate, zirconium dioxide or titanium dioxide. It is preferred that the largest particle size be under 500$\mu$m. If inorganic fillers are used, it is desirable that they first be given surface treatment, e.g. with silane compounds such as $\gamma$-methacryloxypropyltrimethoxysilane, vinyltrichlorosilane, vinyltriethoxysilane, vinyltrimethoxysilane, vinyltriacetoxysilane or vinyltri(methoxyethoxy)silane; silanification may be carried out in a conventional manner. The weight of these fillers is preferably 1 to 7 times the weight of the monomers.

The compositions of the present invention may, if necessary and desired, also include such additives as polymerization inhibitors, colouring agents and UV absorbers. The above-mentioned compositions may be generally supplied to dentists by premixing them in a paste or a solution and packing in a shielded container by the supplier; if necessary and desired, however, the compositions may be packed in two containers. Dentists may apply the compositions of the present invention to the tooth and then polymerize and harden them by visible light from an irradiator in a conventional manner.

As described above, compositions of the present invention comprising the acidic-group-containing monomers can be hardened at a rapid rate by incorporating the above-mentioned specific photopolymerizable initiators. In addition, the compositions are excellent in stability during storage and the hardened materials are free from colouring. Thus, such compositions have excellent properties as compared to conventional photopolymerizable initiators. The compositions of the present invention are used as dental restorative materials, including not only composite materials for filling and restoring a tooth cavity but also inter alia dental crown materials, artificial teeth, dental adhesive agents, dental cementing materials and filling materials for preventing caries.

It is particularly preferred to use the compositions of the present invention as dental adhesives coated on the surface of a tooth cavity prior to filling. In this case, the composition of the present invention is firstly coated onto the surface of the tooth cavity which may or may not have been etched, e.g. with phosphoric acid, and then the cavity is filled with conventional dental filling materials. Hardening of the compositions of the present invention is carried out by irradiating with visible light after coating the compositions of the present invention and/or filling with filling materials.

The dental filling materials used together with the adhesives comprising the compositions of the present invention are generally composed of 10 to 80 wt% (preferably 15 to 50 wt%) of polymerizable monomers and 90 to 20 wt% (preferably 85 to 50 wt%) of fillers. As the polymerizable monomers, the above-mentioned polymerizable monomers copolymerizable with the monomers containing acidic groups are used. Of these, the above-mentioned (meth)acrylic acid esters are preferred.

Further, a variety of fillers (e.g. quartz powders) as described above are used as the fillers. As initiators for the dental filling materials, redox catalysts composed of organic peroxides and amines of the type hardened at room temperature can be used. Alternatively, photoinitiators such as camphorquinone-amines disclosed in U.S. Patent Specification US-A-4 071 424 can be used. If polymerizable monomers having

acidic groups are incorporated in polymerizable monomers for dental filling materials, it is necessary that the photoinitiators used in accordance with the present invention be incorporated. As described above, the adhesive is photohardenable and it is therefore desirable that the initiators for the dental filling materials also be photohardenable.

If the polymerizable monomers hardened by the photoinitiators for the dental filling materials contain no acidic group in the molecule, conventionally known photoinitiators disclosed above in U.S. Patent Specification US-A-4 071 424 can be used; however, it is preferred to use the photoinitiators disclosed in our prior Japanese Patent Application 6797/84 (Japanese Patent Publication (Kokai) 149603/85) which comprise $\alpha$-diketones and derivatives thereof as photosensitizers, and aldehydes free of amino groups and derivatives thereof as accelerators; it is preferred to additionally incorporate organic peroxides such as benzoyl peroxide in them. The photoinitiators disclosed in the said prior application can also harden the polymerizable monomers having acidic groups; however, the photoinitiators used in the present invention are more effective as initiators capable of photohardening such monomers.

In the photoinitiators disclosed in the prior Japanese application, $\alpha$-diketones, quinones and derivatives thereof are the same as the photosensitizers used in the present invention and are selected from the compounds as described above. The aldehydes and their derivatives thereof are preferably compounds of general formula: $B \leftarrow (CHO)_n$, where n is 1, 2 or 3 and B is an acyclic or cyclic saturated or unsaturated aliphatic $C_{1-20}$ hydrocarbon group or a monocyclic or polycyclic aromatic $C_{1-20}$ hydrocarbon group, optionally having a substituent having 1 to 20 carbon atoms, e.g. a $C_{1-20}$ alkylmono- or di-aldehyde; polyalkyl ether mono- or di-aldehydes having 1 to 20 carbon atoms; or compounds represented by the general formula:

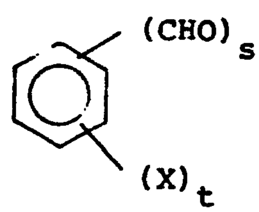

where s is 1 or 2, X represents an alkyl, alkoxy or polyalkyl ether group having 1 to 20 carbon atoms, and t is 0, 1, 2 or 3.

When the compositions of the present invention are used as dental adhesives, restoration of the tooth is effected in combination with the above-mentioned dental filling materials. For this reason, it is preferred that, for convenience, dentists be provided with a set comprising the dental adhesive and the dental filling material in combination.

The present invention will be explained in more detail with reference to the following examples, which are ilustrative but not limiting.

Example 1

A monomer mixture composed of 10 parts by weight of $\beta$-methacryloyloxyethylphenyl phosphoric acid:

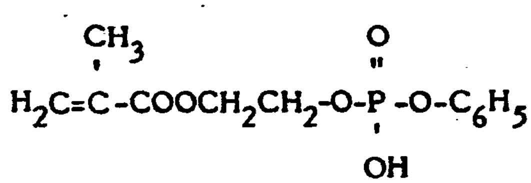

(hereafter referred to as Phenyl-P-monomer), 30 parts by weight of Bis-GMA, 30 parts by weight of HEMA and 30 parts by weight of NPG was prepared and camphorquinone was dissolved in the resulting monomer mixture in a concentration of 0.96 wt%. To the solution were added various thiols shown in Table 1 to prepare respective compositions.

The light source used was a Translux 15 V - 150 W halogen lamp made by Kulzer & Co. GmbH in Germany. Light of wavelength longer than 500 nm was removed through a filter. The monomer composition

was placed in a cylindrical glass sample tube having a thickness of 0.8 mm. This was located 3.2 mm from the top and was irradiated from below. The hardening time was determined by inserting a thermocouple into the monomer mixture and measuring the change in temperature. The point of time when increase of temperature due to polymerization heat ceased (the summit of the peak) was deemed to be the hardening time. The results are shown in Table 1.

Table 1

| Example | -SH group-containing Compound and Addition Amount Thereof (mg) | | Weight of Solution (mg) | Hardening Time (min) |
|---|---|---|---|---|
| 1-1 | None (Control) | | 242.3 | >10 unhardened |
| 1-2 | $CH_3-(CH_2)_{10}-CH_2SH$ | 3.2 | 220.5 | 2.67 |
| 1-3 | $HO-CH_2CH_2-SH$ | 4.2 | 243.5 | 2.17 |
| 1-4 | $HOOC-CH_2CH_2-SH$ | 3.3 | 227.4 | 2.25 |
| 1-5 | $HS-CH_2-CH_2-SH$ | 3.1 | 233.8 | 2.00 |
| 1-6 | 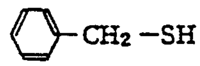 | 5.1 | 224.8 | 2.00 |
| 1-7 | 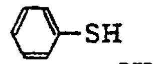 | 2.2 | 229.3 | 1.58 |
| 1-8 | 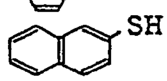 | 4.2 | 237.2 | 1.78 |
| 1-9 | 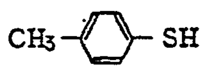 | 2.2 | 229.3 | 1.25 |
| 1-10 | 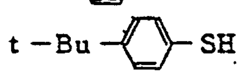 | 1.9 | 234.2 | 0.50~1.00 |
| 1-11 | 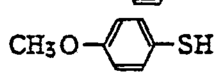 | 3.6 | 230.2 | 2.05 |
| 1-12 | 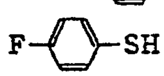 | 4.1 | 224.1 | 1.67 |
| 1-13 | 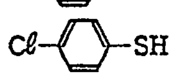 | 5.0 | 222.8 | 1.83 |
| 1-14 | 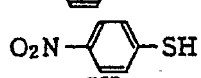 | 3.0 | 227.7 | 1.00 |
| 1-15 | 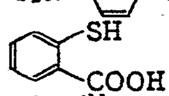 | 2.9 | 219.6 | 1.08 |
| 1-16 | 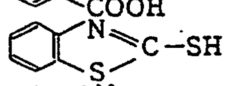 | 2.6 | 219.3 | 0.56 |
| 1-17 | 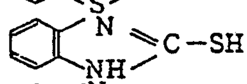 | 2.4 | 223.4 | 0.63 |
| 1-18 | 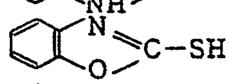 | 3.7 | 235.5 | 0.50 |
| 1-19 | 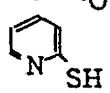 | 4.1 | 226.5 | 0.88 |
| 1-20 | 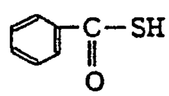 | 5.3 | 226.9 | 0.55 |
| 1-21 | 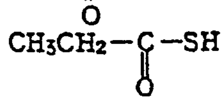 | 4.8 | 233.3 | 0.67 |

Example 2

A composition composed of 9.9 parts by weight of Phenyl-P-monomer, 29.7 parts by weight of HEMA, 29.7 parts by weight of Bis-GMA, 29.7 parts by weight of NPG and 1.0 part by weight of 2-mercaptobenzimidazole was prepared and, various photosensitizers shown in Table 2 were added to the composition, respectively. In a manner similar to Example 1, visible light was exposed to each of the resulting compositions to determine hardening time (only Example 2-1 is in accordance with the invention).

Table 2

| Example | Photoinitiator | Concentration of Photoinitiator (wt%) based on composition | Hardening Time (minute) |
|---|---|---|---|
| 2-1 | Camphorquinone | 0.97 | 0.63 |
| 2-2 | Acenaphthenequinone | 1.00 | 0.95 |
| 2-3 | 1,2-Benzanthraquinone | 0.97 | 0.88 |
| 2-4 | 2-Methylanthraquinone | 1.04 | 0.95 |
| 2-5 | Heptanedione | 1.96 | 1.50 |
| 2-6 | Benzyl | 1.97 | 1.63 |
| 2-7 | Phenanthraquinone | 0.10 | 2.00 |

Example 3

A solution was prepared by dissolving camphorquinone, in a concentration of 1.00 wt%, in a monomer mixture composed of 10 parts by weight of C-10 P monomer:

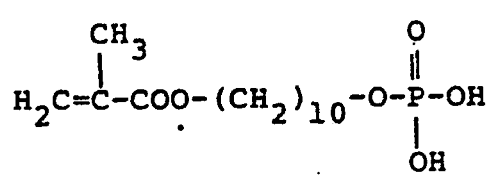

30 parts by weight of Bis-GMA, 30 parts by weight of HEMA and 30 parts by weight of NPG. To the solution were added various thiols shown in Table 3 to prepare respective compositions and the resulting compositions were exposed to light to determine hardening-times. The results are shown in Table 3.

Table 3

| Example | -SH Compound and Addition Amount Thereof (mg) | | Weight of Solution (mg) | Hardening Time (min) |
|---|---|---|---|---|
| 3-1 | Control | | 224.3 | 9.00 |
| 3-2 | 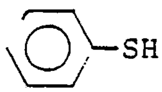 | 3.2 | 237.0 | 0.75 |
| 3-3 | 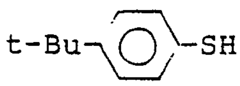 | 3.3 | 233.6 | 1.03 |
| 3-4 | 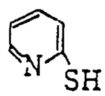 | 1.6 | 239.6 | 0.58 |
| 3-5 | 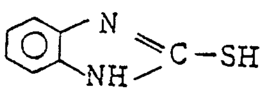 | 3.1 | 226.5 | 0.25 |
| 3-6 | 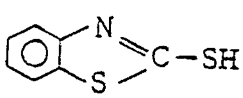 | 2.8 | 233.7 | 0.47 |
| 3-7 | 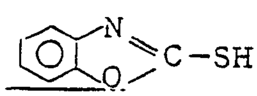 | 3.4 | 226.3 | 0.40 |

Example 4

A composite resin was prepared according to the following composition.

Composition:

| | |
|---|---|
| Phenyl-P-monomer | 0.1138 g |
| TMM-3L | 0.2720 g |
| Epoxy ester 3002 | 0.8161 g |
| Camphorquinone | 0.006 g |
| Mercaptobenzoxazole | 0.0122 g |
| Silane treated quartz powder | 3.9210 g |
| Colloidal silica | 0.050 g |

These various components were kneaded in a mortar to prepare a paste. After the paste was degassed in vaccum in a desiccator, the paste was filled up in a glass tube having an inner diameter of 3 mm in a length of 12 mm. From one side of the glass tube, light was irradiated for 10 seconds at a distance of 4 mm using the light source described in Example 1. Thereafter a needle having a diameter of 1 mm was inserted from the other side of the glass tube to which no light was exposed and a load of 260 g was applied to the needle for 30 seconds to measure the degree the needle penetrated, whereby the thickness of the hardened portion was measured. The composite resin of 7 mm was hardened.

Example 5

A bonding agent and a composite resin were prepared according to the following compositions.

Composition 1: Bonding Agent

| | | |
|---|---|---|
| Bis-GMA | 30 | parts by weight |
| NPG | 30 | parts by weight |
| HEMA | 30 | parts by weight |
| Phenyl-P-monomer | 10 | parts by weight |
| Camphorquinone | 1.5 | part by weight |
| Mercaptobenzothiazole | 1.5 | part by weight |

Composition 2: Composite Resin

| | | |
|---|---|---|
| Bis-GMA | 17.33 | parts by weight |
| 3G | 5.77 | parts by weight |
| Camphorquinone | 0.34 | part by weight |
| Mercaptobenzothiazole | 0.34 | part by weight |
| Silane treated quartz powder | 73.96 | parts by weight |
| Colloidal silica | 2.26 | parts by weight |

The thus prepared bonding agent and the composite resin were combined together to determine adhesion force to ivory bars. Adhesion test to the ivory bars was performed by the following method. First, the edge surfaces of two ivory bars (10 x 10 x 50 mm) were abraded with emery paper. After rinsing with water, the bonding agent was thinly coated on to the abraded surfaces using a small brush. Then, a paste of the composite resin was put in a layer onto the surface coated with the bonding agent, using a CR-syringe, which was exposed to light for 2 minutes using the light source described in Example 1. Next, the photohardened resin portion was stuck to the other ivory bar using a conventional dental composite resin (registered trademark: Clearfill FII). After soaking it in water at 37°C overnight, it was subjected to a tensile test using Instron tensile tester. The adhering matter showed an adhesive force of 47.6 $kg/cm^2$.

Comparison Example 1

A solution was prepared by dissolving camphorquinone (0.97 wt%) and N,N-dimethyl-p-toluidine (0.99 wt%) in a monomer mixture composed of 30 parts by weight of Bis-GMA, 30 parts by weight of NPG and 40 parts by weight of HEMA. Phenyl-P-monomer was added to the solution in an amount shown in Table 4 to obtain a composition. The composition was exposed to light to measure a hardening time. For purpose of comparison, the results obtained by adding no Phenyl-P-monomer are also shown.

Table 4

| Comparison Example | Weight of Phenyl-P-monomer (mg) | Weight of Solution (mg) | Hardening Time (sec) |
|---|---|---|---|
| 1-1 | 0 | 227.4 | 68 |
| 1-2 | 27 | 220 | 200 |

Comparison Example 2

A solution was obtained by dissolving camphorquinone (0.90 wt%) and benzoyl peroxide (0.79 wt%) in the same monomer mixture as in Comparison Example 1 and, methacrylic acid was added thereto in an amount described in Table 6. The resulting composition was exposed to light to measure a hardening time. For purpose of comparison, the results obtained by adding no methacrylic acid are also shown.

Table 5

| Comparison Example | Weight of Methacrylic Acid (mg) | Weight of Solution (mg) | Hardening Time (sec) |
|---|---|---|---|
| 2-1 | 0 | 224.1 | 51 |
| 2-2 | 10.1 | 218 | 210 |

Comparison Example 3

A solution was prepared by dissolving 0.06 wt% of β-naphthoquinone in a monomer mixture composed of 9.47 parts by weight of Phenyl-P-monomer, 22.63 parts by weight of pentaerythritol trimethacrylate and 67.90 parts by weight of epoxy ester 3002 and, amines shown in Table 6 were added thereto. The resulting compositions were exposed to light to measure hardening times. The results are shown in Table 6.

Table 6

| Comparison Example | Kind of Amine and Addition Amount Thereof (mg) | | Amount of Monomer Mixture (mg) | Hardening Time (min) |
|---|---|---|---|---|
| 3-1 | None (control) | | 231.0 | 4.00 |
| 3-2 | Triethanolamine | 7.5 | 273.3 | 4.00-5.00 |
| 3-3 | N,N-Dimethyl-aminoethyl methacrylate | 6.5 | 219.2 | 3.67-4.00 |
| 3-4 | Triethylamine | 15 | 248.0 | 4.00-4.33 |

Example 6

A bonding agent and a composite resin were prepared according to the following compositions.

Composition 3: Bonding Agent

| | |
|---|---|
| Bis-GMA | 50 parts by weight |
| C-10 P monomer | 5 parts by weight |
| NPG | 15 parts by weight |
| HEMA | 30 parts by weight |

| | |
|---|---|
| Camphorquinone | 1 part by weight |
| Mercaptobenzoxazole | 1 part by weight |

Composition 4A: Composite Resin

| | |
|---|---|
| Bis-GMA | 10 parts by weight |
| 3G | 4 parts by weight |
| Camphorquinone | 0.1 part by weight |
| Benzoyl peroxide | 0.15 part by weight |
| p-Tolualdehyde | 0.3 part by weight |
| Silane treated quartz powder | 79 parts by weight |
| Colloidal silica | 4 parts by weight |

Composition 4B: Composite Resin

| | | |
|---|---|---|
| Bis-GMA | 10 | parts by weight |
| 3G | 4 | parts by weight |
| Camphorquinone | 0.1 | part by weight |
| N,N-Dimethylamino-ethyl methacrylate | 0.3 | part by weight |
| Silane treated quartz powder | 79 | parts by weight |
| Colloidal silica | 4 | parts by weight |

The thus obtained bonding agent and the composite resin A or B were combined together to determine adhesion force to ivory bars. The ivory bars were treated in a manner similar to that of Example 5. After the bonding agent had been thinly coated onto the edge surfaces of the ivory bars, about 0.3 g of a paste of the composite resin was put on the coated surfaces and the paste was spread over the whole edge surfaces of the ivory bars using a glass piece to form a paste layer having a thickness of 1 mm. Thereafter, light was irradiated for 40 seconds using the light source described in Example 1. About 5 minutes after, the glass piece was stripped off from the hardened resin and, another ivory bar was stuck to the hardened resin using commercially available dental composite resin (registered trademark, Clearfill FII). After soaking it in water at 37° C overnight, adhesive force was measured using Instron tensile tester. Compositions A and B gave adhesion strengths of 130 kg/cm$^2$ and 90 kg/cm$^2$, respectively.

Example 7

Using the bonding agent having Composition 3 prepared in Example 6 and a composite resin (paste

type) having the following composition, the adhesive force to ivory bars was determined.

Composition 5:

| | | |
|---|---|---|
| Bis-GMA | 10 | parts by weight |
| 3G | 4 | parts by weight |
| Benzoyl peroxide | 0.25 | part by weight |
| Silane treated quartz powder | 79 | parts by weight |
| Colloidal silica | 4 | parts by weight |

Composition 6

| | | |
|---|---|---|
| Bis-GMA | 10 | parts by weight |
| 3G | 4 | parts by weight |
| N,N-diethanol-p-toluidine | 0.12 | part by weight |
| Silane treated quartz powder | 79 | parts by weight |
| Colloidal silica | 4 | parts by weight |

Two ivory bars treated in a manner similar to that of Example 6 were prepared. At the edge of each of the bars, a bonding agent having Composition 3 was thinly coated and light was irradiated for 20 seconds using Translux to harden the above-mentioned bonding agent. Then, a paste obtained by kneading composite resins of Compositions 5 and 6 was put on the hardened portion. Thereafter two ivory bars were brought into close contact and allowed to stand for 10 minutes at 25° C. After soaking in water at 37° C overnight, adhesive force was measured using an Instron tensile tester. Adhesive strength of 80 kg/cm$^2$ was obtained.

Comparison Example 4

Allylthiourea, which is used as a photoaccelerator for a dental composition containing an acidic-group-containing monomer of Japanese Laid Open Patent Application 56-120610, was added to Composition 3 of Example 6, in place of mercaptobenzoxazole. The thus obtained bonding agent and the composite resin A of Example 6 were combined together to determine the adhesive force to ivory bars. The adhesive force was measured in the same manner as in Example 6. The composition gave an adhesive strength of 70 kg/cm$^2$.

## Claims

1. A dental composition comprising a vinyl monomer containing at least one acidic group in the molecule and an initiator capable of photopolymerizing the monomer by visible light, characterized in that the initiator comprises camphorquinone as a photosensitizer, together with an accelerator that contains at least one mercapto group in the molecule and does not contain an aldehyde group in the molecule.

2. A composition according to claim 1 in which the accelerator is of the general formula B - SH in which B represents a $C_{1-20}$ aliphatic hydrocarbon or $C_{6-20}$ aromatic hydrocarbon group optionally having alkyl,

aryl, hydroxyl, alkoxy, carboxyl, alkoxycarbonyl, acyl, dialkylamino, amino, mercapto, halogen, carbamoyl, or nitro substitution in which the alkyl, aryl, alkoxy, alkoxycarbonyl, acyl and dialkyl amino groups have up to 20 carbon atoms.

**3.** A composition according to claim 1 in which the accelerator is of the general formula

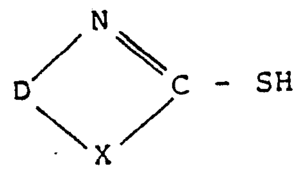

where D forms a 5- or 6-membered ring together with the

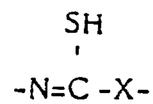

residue and is bound through a single bond or a double bond or is composed of 2 or 3 carbon atoms forming a part of one aromatic ring, or is composed of one nitrogen atom bound to one carbon atom; and X represents -O-, -S-,

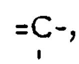

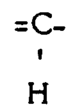

or NR where R is H or $C_{1-4}$ alkyl.

**4.** A composition according to claim 3 in which the accelerator is 2-mercaptobenzoxazole, 2-mercaptobenzimidazole or 2-mercaptobenzothiazole.

**5.** A composition according to claim 1 in which the accelerator is of the general formula

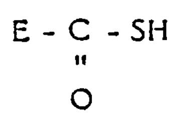

where E represents a phenyl group, which optionally contains a $C_{1-20}$ alkyl substituent, or a $C_{1-20}$ alkyl group.

**6.** A dental composition according to any one of claims 1 to 5 in which the acidic group in the vinyl monomer is a

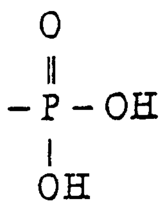 , 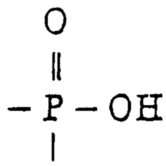 , 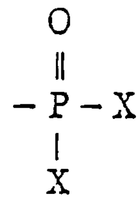 , 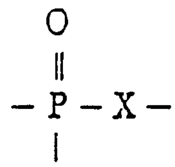 ,

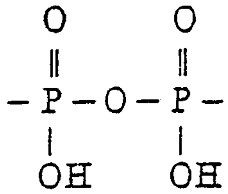 , 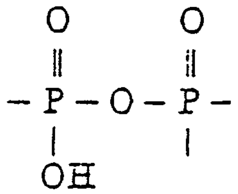 , $-COOH$ , 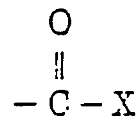

or

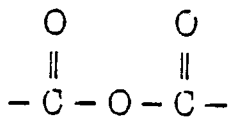

group, X being F, Cl, Br or I.

7. A composition according to any one of claims 1 to 6 including a vinyl monomer copolymerizable with the vinyl monomer containing at least one acidic group.

8. A composition according to any one of claims 1 to 7 that also contains a filler.

9. A kit for restoration of a tooth comprising a combination of (1) a dental composition comprising a vinyl monomer containing at least one acidic group in the molecule and an initiator and an acccelerator as defined in claim 1, and (2) a dental filling composition comprising a polymerizable monomer, an initiator capable of polymerizing the monomer and a filler.

10. A kit according to claim 9 in which the accelerator in (2) is an aldehyde containing no amino group or a derivative thereof.

11. A kit according to claim 9 or 10 in which the initiator in (2) is a redox type initiator consisting essentially of an organic peroxide and an amine.

**Revendications**

1. Une composition dentaire comprenant un monomère vinylique contenant au moins un groupe acide dans sa molécule et un amorceur capable de photopolymériser le monomère sous l'action de la lumière visible, caractérisée on ce que l'amorceur comprend de la camphoquinone comme photosensibilisateur, avec un accélérateur qui contient au moins un groupe mercapto dans sa molécule et qui ne contient pas un groupe aldéhyde dans sa molécule.

2. Une composition selon la revendication 1, dans laquelle l'accélérateur répond à la formule générale B-SH dans laquelle B représente un groupe hydrocarboné aliphatique en $C_{1-20}$ ou un groupe hydrocarboné aromatique en $C_{6-20}$, ayant facultativement une substitution alcoyle, aryle, hydroxyle, alcoxy, carboxyle, alcoxycarbonyle, acyle, dialcoylamino, amino, mercapto, halogène, carbamoyle ou nitro, où les groupes alcoyle, aryle, alcoxy, alcoxycarbonyle, acyle et dialcoylamino ont jusqu'à 20 atomes de

carbone.

3. Une composition selon la revendication 1, dans laquelle l'accélérateur répond à la formule générale

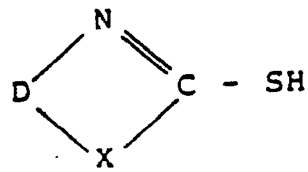

dans laquelle D forme un cycle à 5 ou 6 chaînons avec le reste

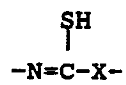

et est lié par une simple liaison ou une double liaison, ou est composé de 2 ou 3 atomes de carbone faisant partie d'un cycle aromatique, ou est composé d'un atome d'azote lié à un atome de carbone ; et X représente -O-, -S-,

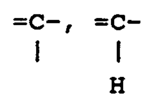

ou NR où R est H ou un alcoyle en $C_{1-4}$.

4. Une composition selon la revendication 3, dans laquelle l'accélérateur est le 2-mercaptobenzoxazole, le 2-mercaptobenzimidazole ou le 2-mercaptobenzothiazole.

5. Une composition selon la revendication 1, dans laquelle l'accélérateur répond à la formule générale

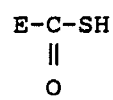

où E représente un groupe phényle, qui contient facultativement un substituant alcoyle en $C_{1-20}$, ou un groupe alcoyle en $C_{1-20}$.

6. Une composition dentaire selon l'une quelconque des revendications 1 à 5, dans laquelle le groupe acide dans le monomère vinylique est un groupe

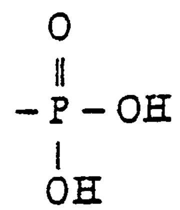 , 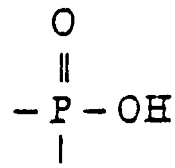 , 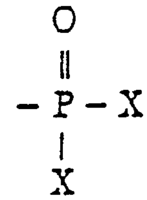 , 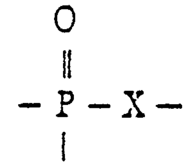 ,

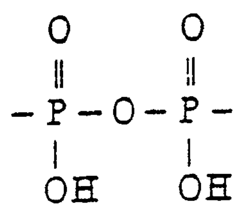 , 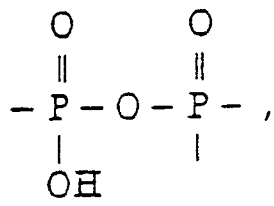 , -COOH , 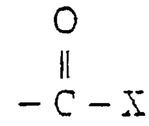

ou

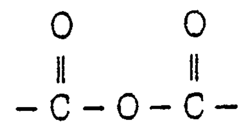 ,

X étant F, Cl, Br ou I.

7. Une composition selon l'une quelconque des revendications 1 à 6 comprenant un monomère vinylique copolymérisable avec le monomère vinylique contenant au moins un groupe acide.

8. Une composition selon l'une quelconque des revendications 1 à 7 qui contient également une charge.

9. Un nécessaire pour la restauration d'une dent comprenant une combinaison de (1) une composition dentaire comprenant un monomère vinylique contenant au moins un groupe acide dans sa molécule et un amorceur et un accélérateur, comme défini dans la revendication 1, et (2) une composition d'obturation dentaire comprenant un monomère polymérisable, un amorceur capable de polymériser le monomère et une charge.

10. Un nécessaire selon la revendication 9, dans lequel l'accélérateur de (2) est un aldéhyde ne contenant pas de groupe amino ou un dérivé de celui-ci.

11. Un nécessaire selon la revendication 9 ou 10, dans lequel l'amorceur de (2) est un amorceur de type redox constitué essentiellement d'un peroxyde organique et d'une amine.

**Patentansprüche**

1. Dentale Zusammensetzung enthaltend ein Vinylmonomer, das mindestens eine saure Gruppe im Molekül enthält, und einen Initiator mit der Fähigkeit, das Monomer bei sichtbarem Licht zu photopolymerisieren, dadurch gekennzeichnet, daß der Initiator Kampferchinon als Photosensibilisator zusammen mit einem Beschleuniger enthält, der mindestens eine Mercaptogruppe im Molekül und keine Aldehydgruppe im Molekül enthält.

2. Zusammensetzung nach Anspruch 1, worin der Beschleuniger die allgemeine Formel B - SH hat, worin B eine $C_{1-20}$ aliphatische Kohlenwasserstoffgruppe oder eine $C_{6-20}$ aromatische Kohlenwasserstoffgruppe bedeutet, die gegebenenfalls mit Alkyl-, Aryl-, Hydroxyl-, Alkoxy-, Carboxyl-, Alkoxycarbonyl-, Acyl-, Dialkylamino-, Amino-, Mercaptogruppen, Halogen, Carbamoyl- oder Nitrogruppen substituiert sind, wobei die Alkyl-, Aryl-, Alkoxy-, Alkoxycarbonyl-, Acyl- und Dialkylaminogruppen bis zu 20 Kohlenstoffatome haben.

3. Zusammensetzung nach Anspruch 1, worin der Beschleuniger die allgemeine Formel

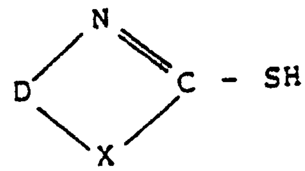

hat, worin D zusammen mit dem

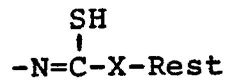

einen 5- oder 6-gliedrigen Ring bildet und über eine Einfach- oder Doppelbindung gebunden ist, oder aus zwei oder drei Kohlenstoffatomen, die einen Teil eines aromatischen Ringes bilden, zusammengesetzt ist, oder aus einem Stickstoffatom, das an ein Kohlenstoffatom gebunden ist, zusammengesetzt ist; und X -O-, -S-,

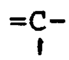, 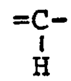

oder NR bedeutet, worin R H oder $C_{1-4}$-Alkyl ist.

**4.** Zusammensetzung nach Anspruch 3, worin der Beschleuniger 2-Mercaptobenzoxazol, 2-Mercaptobenzimidazol oder 2-Mercaptobenzothiazol ist.

**5.** Zusammensetzung nach Anspruch 1, worin der Beschleuniger die allgemeine Formel

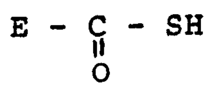

hat, worin E eine Phenylgruppe, die gegebenenfalls einen $C_{1-20}$-Alkylsubstituenten enthält oder eine $C_{1-20}$-Alkylgruppe bedeutet.

**6.** Dentale Zusammensetzung nach einem der Ansprüche 1 bis 5, worin die saure Gruppe im Vinylmonomer

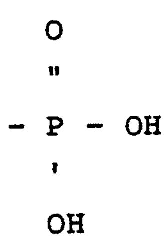, 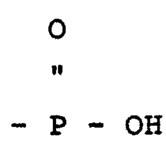, 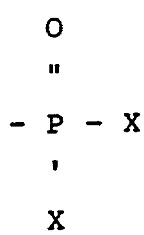, 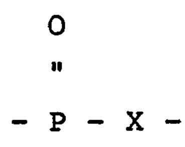,

$$-\underset{OH}{\overset{O}{\overset{\|}{P}}}-O-\underset{OH}{\overset{O}{\overset{\|}{P}}}-,\quad -\underset{OH}{\overset{O}{\overset{\|}{P}}}-O-\underset{|}{\overset{O}{\overset{\|}{P}}}-,\quad -COOH,\quad -\overset{O}{\overset{\|}{C}}-X$$

oder eine

$$-\overset{O}{\overset{\|}{C}}-O-\overset{O}{\overset{\|}{C}}-$$

Gruppe ist, wobei X F, Cl, Br oder I ist.

7. Zusammensetzung nach einem der Ansprüche 1 bis 6, die ein Vinylmonomer enthält, das mit dem Vinylmonomer mit mindestens einer sauren Gruppe copolymerisierbar ist.

8. Zusammensetzung nach einem der Ansprüche 1 bis 7, die zudem einen Füllstoff enthält.

9. Set zur Wiederherstellung von Zähnen, enthaltend eine Kombination aus (1) einer dentalen Zusammensetzung enthaltend ein Vinylmonomer, das mindestens eine saure Gruppe im Molekül enthält, und einen Initiator und einen Beschleuniger wie in Anspruch 1 definiert, und (2) einer dentalen Füllzusammensetzung, enthaltend ein polymerisierbares Monomer, einen Initiator mit der Fähigkeit, das Monomer zu polymerisieren und einen Füllstoff.

10. Set nach Anspruch 9, worin der Beschleuniger in (2) ein Aldehyd, der keine Aminogruppe enthält, oder ein Derivat davon ist.

11. Set nach Anspruch 9 oder 10, worin der Initiator in (2) ein Initiator vom Redoxtyp ist, der im wesentlichen aus einem organischen Peroxid und einem Amin besteht.